# EUROPEAN PATENT SPECIFICATION

(11) **EP 2 244 986 B1**
(45) Date of publication and mention of the grant of the patent: **15.02.2012**
(21) Application number: 08858113.7
(22) Date of filing: 04.12.2008
(51) Int. Cl.: C02F 11/04, C02F 1/00, C02F 1/36, B02C 19/18, C02F 1/34

(54) **METHOD FOR TREATMENT OF ORGANIC MATERIAL**
VERFAHREN ZUR BEHANDLUNG VON ORGANISCHEM MATERIAL
PROCÉDÉ DE TRAITEMENT DE MATIÈRE ORGANIQUE

(30) Priority: 06.12.2007 SE 0702721
(43) Date of publication of application: 03.11.2010
(73) Proprietor: Scandinavian Biogas Fuels AB, 111 22 Stockholm (SE)
(72) Inventor: EJLERTSSON, Jörgen, 590 46 Rimforsa (SE); HÖGSTRÖM, Jonas, S-743 40 Storvreta (SE); EASINGWOOD, Mark, S-755 95 Uppsala (SE)
(74) Representative: Stenborg, Anders Vilhelm
(86) International application number: PCT/EP2008/010274
(87) International publication number: WO 2009/071294

(56) References cited:
- WO-A-98/08774
- DE-A1- 10 229 363
- GB-A- 2 419 877
- US-A- 5 129 469
- US-A- 6 045 070

## Description

### Technical field

The present invention relates to a method for treatment of organic material, especially when producing biogas using anaerobic digestion of the organic material, and to a system for treatment of organic material according to the method.

### Background

It is well known in the prior art that ultrasonic treatment of organic material will decrease the viscosity with maintained dry solid contents, whereby a higher degree of dry solid contents may be obtained, e.g. in a feed line inputting organic material into a conventional biogas producing reactor tank, such as described the international publication WO 2004/016796 A1, assigned to Tekniska Verken i Linköping AB. Ultrasonic reactors are available from several manufacturer and distributors, such as Ultra "Sonus, Sonica, Ultrawaves and Purac.

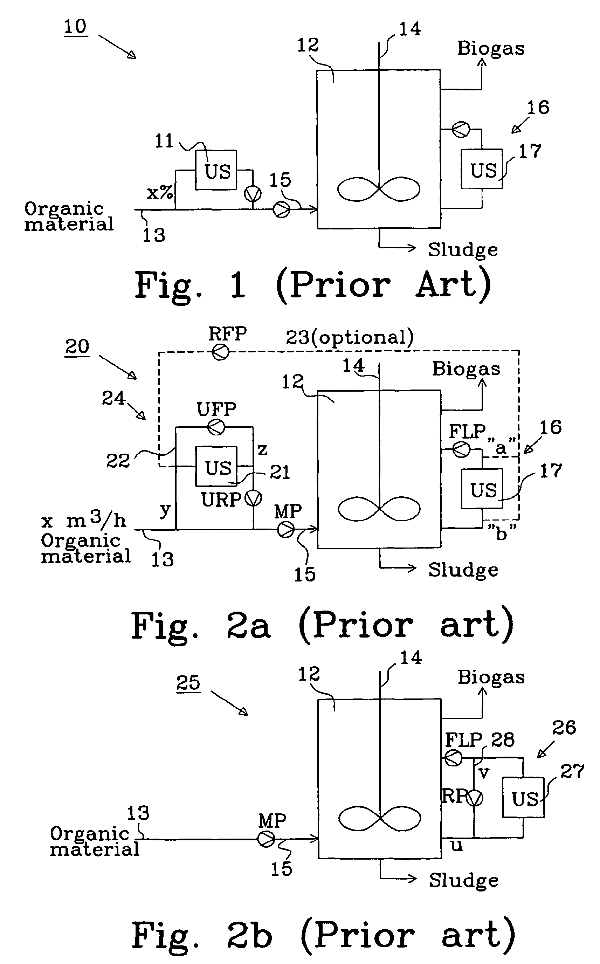

Ultrasonic treatment of organic material in a biogas producing system is presently used to increase the amount of biogas that may be produced. The organic material is mechanical broken before it is introduced into the reactor. The capacity of prior art ultrasonic equipment is rather limited, which makes it difficult to treat all the organic material before it is introduced into the reactor. Figure 1 shows a prior art system 10 provided with two independent prior art systems for treating organic material when producing biogas in a tank reactor under anaerobic condition. In the first prior art system only x% (approx 30-40 %) of an organic material introduced by a pipe 13 is subject to ultrasonic treatment in an ultrasonic reactor 11 before it is transported to a reactor tank 12. The dry solids contents is limited to 2-3% by weight TS, and the energy consumption needed to perform the ultrasonic treatment is approximately 4W/h per litre. It is, however, possible to use several ultrasonic reactors in parallel but the amount of energy needed would result in a net loss of energy for the complete biogas producing process. Thus, the organic material is only partly treated by the ultrasonic reactor 11, and thereafter introduced through a feed pipe 15 into the reactor tank 12.

The limited capacity of the ultrasonic reactor makes it impossible to treat all the organic material before introducing it into the reactor which is a drawback. The second prior art system 16, e.g. disclosed in the international publication WO 2005/016829, comprises a feedback loop including an ultrasonic reactor 17. Sludge from the reactor tank 12 is pumped and introduced into the ultrasonic reactor 17 for ultrasonic treatment, and is thereafter reintroduced into the reactor tank 12. Increased surface area of the organic material is achieved to increase the biogas production. Internal mixing is provided in the reactor tank 12, as exemplified by an agitator 14.

JP 2006-122875 discloses an equipment for sludge solubilisation treatment including a feedback loop from the outlet of an ultrasonic reactor to the inlet of the same ultrasonic reactor. At least a portion of the material available at the outlet is fed back to the inlet in order to decrease the viscosity of the material introduced. The capacity of the treatment equipment is increased compared to the prior art system described in WO 2005/016829.

US 5,129,469 discloses removal of drill cuttings from drilling fluid. The cuttings are conveyed to a shearing and grinding system that converts the cuttings into a viscous slurry with the addition of water. The system comprises a receiving tank and a centrifuging pump for recirculating the mixture of cuttings and water between the pump and the receiving tank. A discharge conduit is connected to the pump for moving the slurry to an injection pump.

US 6,045,070 discloses a series of solids size reduction systems including infeed, receiving and mixing systems for blending solids and carrier fluids to a grinding apparatus as well as separation and recover of the carrier fluids for recycling.

### Summary of the invention

An object with the present invention is to provide a system and a method for disintegrating at least one organic material to control the viscosity of the organic material while maintaining high dry solids contents of the organic material compared to the prior art.

The object is achieved by the system of claim 1 and the method of claim 8 comprising at least two disintegrating nits, such as ultrasonic reactors or mechanical dispersion devices, each provided with an inlet for receiving organic material, and an outlet for outputting disintegrated organic material. A first feedback pipe is connected B, C is available at the outflow 39; 49; 64.

A first feedback pipe is connected between the outlet of one of the disintegrating units, which also is connected to an outflow of the system, and the inlet of another.

In a preferred embodiment, a second feedback pipe is provided to feedback the disintegrated organic material at the outlet of the another disintegrating unit to the inlet of the disintegrating unit being connected to the outflow.

An advantage with the present invention is that different organic material may be mixed together more easily for instance before it is introduced into a biogas producing system.

Another advantage of the present invention is that organic material with low dry solids contents may be subject to ultrasonic treatment compared to prior art systems.

Another advantage and aspect of the present invention is that an increased amount of biogas may be produced in an anaerobic digestion process for producing biogas compared to prior art systems.

Further objects and advantages will be apparent for a skilled person from the detailed description and the accompanying drawings.

### Brief description of the drawings

Figure 1 shows a system for producing biogas from organic material with ultrasonic treatment according to prior art.
Figure 2a shows a variant of an ultrasonic system for treatment of organic material before being fed into a biogas producing system according to prior art.
Figure 2b shows another variant of an ultrasonic system for treatment of organic material in a feedback loop arranged to a biogas producing system according to prior art.
Figure 3 shows a first embodiment of a disintegrating system in which more than one type of organic material may be treated.
Figures 4 shows a second embodiment of a disintegrating system in which more than one type of organic material may be treated.
Figures 5 shows a third embodiment of a disintegrating system in which more than one type of organic material may be treated.
Figure 6 shows a first embodiment of a biogas producing system including disintegrating systems according to the invention.
Figure 7 shows a second embodiment of a biogas producing system including disintegrating systems according to the invention.
Fig. 8 shows an alternative embodiment of a biogas producing system in which material with different properties, such as different temperature or dry solids contents, are mixed before introduced into a disintegrating system.

### Detailed description of preferred embodiments

Figure 1 discloses a biogas producing system 10 provided with two independent ultrasonic treatment system according to prior art, as described in more detail above.

Figure 2a discloses a biogas producing system 20 provided with a variant of a prior art ultrasonic treatment system 24 for treatment of organic material. An amount ("x" m³/h) of organic material is fed into a pipe 13 and an ultrasonic reactor pump (URP) feeds a part "y" of the organic material fed into pipe 13 to an inlet of an ultrasonic reactor 21. The URP controls the amount, which preferably corresponds to 30-100% of "x", and the remaining part of the organic material, preferably 0-70%, is directly introduced into the reactor tank 12 through the feed pipe 15. A feedback loop 22 is provided from an outlet of the ultrasonic reactor 21 to the inlet of the ultrasonic reactor 21. An amount "z" of the ultrasonic treated organic material present at the outlet is introduced into the feedback loop 22 using an ultrasonic feedback pump (UFP). The UFP controls the amount, which preferably corresponds to up to 1000% of "y", more preferably 100-300% of "y". The dry solid contents of the ultrasonic treated organic material is up to 6-8% TS. The outlet of the ultrasonic reactor is also connected to the inlet of the reactor tank 12 through the feed pipe 15. A main pump (MP) controls the amount of organic material "x" fed into the reactor tank 12, and thus the amount of organic material fed into the pipe 13.

The biogas producing system 20 is further provided with a prior art ultrasonic treatment system 16, as described in connection with figure 1, having a feedback loop including an ultrasonic reactor 17 and a feedback loop pump (FLP). Digested organic material, or sludge, from the reactor tank 12 is pumped, or let out using a valve (not shown), and introduced into the ultrasonic reactor 17 for ultrasonic treatment using the FLP, and is thereafter reintroduced into the reactor tank 12. An optional return feed pipe 23 (dashed line) may be provided, instead of the feedback loop 22, between the outlet of the ultrasonic reactor 17 (position "a") and the inlet of the ultrasonic reactor 21. A return feed pump RFP may be provided. Ultrasonic treated sludge may then be returned and mixed with freshly introduced organic material provided through pipe 13. The feedback loop 22 may be provided together with the return feed pipe 23, whereby ultrasonic treated organic material provided through the feedback loop 22 is introduced into the ultrasonic reactor 21 together with organic material through pipe 13 and ultrasonic treated organic material from the ultrasonic reactor 17.

A constant flow over the ultrasonic reactor 21 is achieved, and the treatment effect is reduced, by circulating digested organic material, or sludge, from the reactor tank. The sludge may be ultrasonic treated, pumped from position "a", or non-ultrasonic treated, pumped from position "b".

Figure 2b shows a second embodiment of an ultrasonic system 26 for treatment of organic material in a feedback loop provided with a ultrasonic reactor 27 arranged to a biogas producing system 25, similar to the prior art system 16 described in connection with figure 1. A feedback loop pump (FLP) is arranged in the feedback loop to pump, or to let out using a valve (not shown), a certain amount of digested organic material "u" from the reactor tank 12 and introduce the same amount of ultrasonic treated organic material into the reactor tank 12. The ultrasonic system 26 further comprises a return pipe 28 between the outlet and the inlet of the ultrasonic reactor 27. A return pump (RP) is provided in the return pipe and configured in such a way that a predetermined amount "v" of the ultrasonic treated organic material is fed back to the inlet of the ultrasonic reactor 27. The RP controls the amount, which preferably corresponds to up to 1000% of "u", more preferably 100-300% of "u".

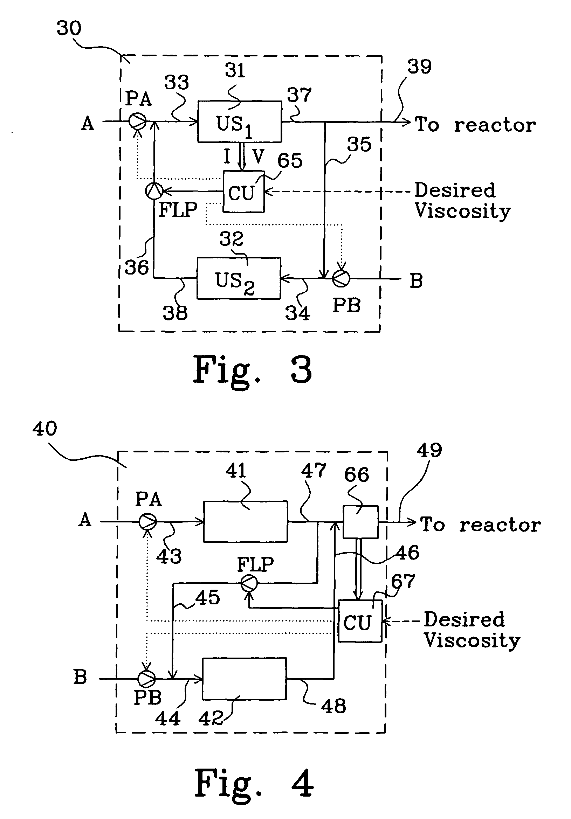

Figure 3 shows a first embodiment of a disintegrating system according to the invention, which in this embodiment is an ultrasonic system 30 comprising two ultrasonic reactors 31 and 32. Organic material "A" is introduced into an inlet 33 of a first ultrasonic reactor 31 (denoted US₁ in figure 3) through a first feeding pipe, and organic material "B", which may differ from organic material "A", is introduced into an inlet 34 of a second ultrasonic reactor 32 (denoted US₂ in figure 3) through a second feeding pipe. Ultrasonic treated organic material is transferred from an outlet 37 of the first ultrasonic reactor US₁ to the inlet 34 of the second ultrasonic reactor US₂ through a first feedback pipe 35, and ultrasonic treated organic material is transferred from an outlet 38 of the second ultrasonic reactor US₂ to the inlet 33 of the first ultrasonic reactor US₁ through a second feedback pipe 36. Ultrasonic treated organic material is accessible for a biogas producing reactor tank at an outflow 39 connected to the outlet 37 of the first ultrasonic reactor US₁.

An advantage with this embodiment is that different organic material may be mixed together more easily for instance before it is introduced into a biogas producing system such as described in connection with figure 2a. This is achieved by controlling the flow through a number of pumps. A first feeding pump PA is provided to introduce organic material "A" into the first ultrasonic reactor 31, and a second feeding pump PB is provided to introduce organic material "B" into the second ultrasonic reactor 32. The capacity of each pump PA and PB is preferably 0-15 m³/h. A feedback loop pump FLP, which is arranged in the feedback pipe 36 has preferably a capacity of 15-30 m³/h.

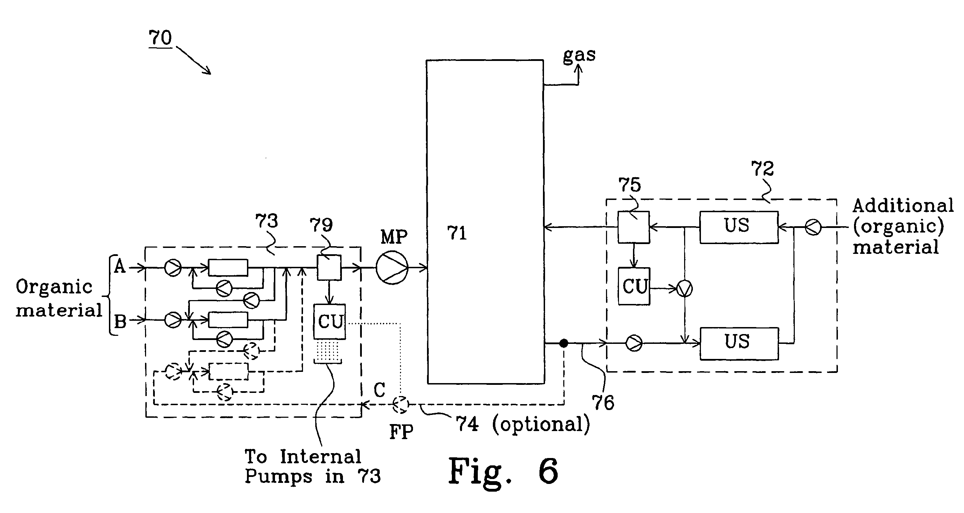

The amount of ultrasonic treated material that is transported to a biogas producing reactor through outflow 39 is controlled by a main pump (not shown) arranged at the inlet of the tank reactor as illustrated in connection with figure 6. If the main pump is set to transport Q m³/h into the biogas producing reactor, the sum of the organic materials A and B introduced through PA and PB should be equal to Q, i.e. A+B=Q. In order to maintain a loop flow, the flow through FLP needs to be higher than Q.

Viscosity is an interesting property of the ultrasonic treated material provided through the outflow 39 that might be desired to monitor. This may be achieved by a viscosity sensor arranged at the outflow 39 connected to a control unit, which in turn controls the flow through the FLP as illustrated in figure 4.

In figure 3, an alternative embodiment is shown wherein the viscosity sensor is omitted and the viscosity of the material transported through the first ultrasonic reactor 31 (and thus the material available at the outflow 39) may be determined by measuring the relationship between the voltage and current supplied to the ultrasonic reactor 31. The relationship between the voltage and current varies as a function of the viscosity of the treated organic material and is analysed in a control unit (CU) 65, which in turn controls the flow through the FLP. It is naturally possible to also control the flow of the first and second feeding pump PA and PB from the CU 65, as indicated by the dotted lines.

Figure 4 shows a second embodiment of a disintegrating system according to the invention, which in this embodiment is a mechanical dispersion system 40 comprising two dispersion devices 41 and 42 arranged in a different way compared to figure 3. Organic material "A" is introduced into a first dispersion device 41 through a first feeding pipe to an inlet 43 of the first dispersion device 41, and organic material "B", which may differ from organic material "A", is introduced into a second dispersion device 42 through a second feeding pipe to an inlet 44 of the second dispersion device 42. Treated organic material is transferred from the first dispersion device 41 to the inlet 44 of the second dispersion device 42 through a feedback pipe 45, and treated organic material is transferred from an outlet 48 of the second dispersion device 42 to an outlet 47 of the first dispersion device 41 through a feed forward pipe 46. Treated organic material is accessible for a biogas producing reactor tank at an outflow 49 connected to the outlet 47 of the first dispersion device 41 and the outlet 48 of the second dispersion device 42.

Treated organic material is provided through the outflow 49 to be accessible to a biogas producing reactor by controlling the flow through a number of pumps. A first feeding pump PA is provided to introduce organic material "A" into the first dispersion device 41, and a second feeding pump PB is provided to introduce organic material "B" into the second dispersion device 42. The capacity of each pump PA and PB is preferably 0-15 m³/h. A feedback loop pump FLP, which is provided in the feedback pipe 45 has in this embodiment preferably a capacity of 0-15 m³/h.

The amount of ultrasonic treated material that is transported to a biogas producing reactor through the outflow 49 is controlled by a main pump (not shown) arranged at the inlet of the tank reactor as illustrated in connection with figure 6. If the main pump is set to transport Q m³/h into the biogas producing reactor, the sum of the organic materials A and B introduced through PA and PB should be equal to Q, i.e. A+B=Q. The flow through FLP will determine how much of the organic material "A" that will be subject to another treatment in the second dispersion device 42.

A viscosity sensor 66 is in this embodiment arranged at the outflow 49 and is connected to a control unit 67, which in turn controls the flow through the FLP. More viscosity sensors may naturally be provided in the dispersion system 40 in order to monitor the viscosity and ultimately to control the flow through each available pump as indicated by the dotted lines.

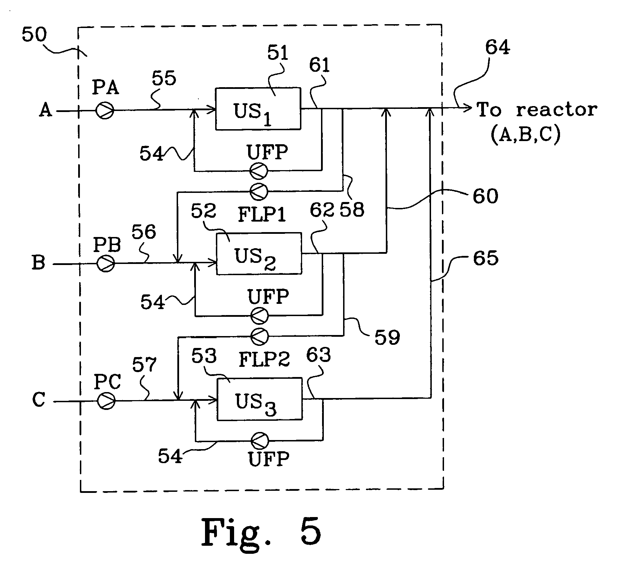

Figure 5 shows a disintegrating system according to the preamble of claim 1, which is an ultrasonic system 50 having a more complex structure. This ultrasonic system 50 comprises three ultrasonic reactors 51, 52 and 53 (denoted US₁, US₂ and US₃, respectively in figure 5). Each ultrasonic reactor is provided with a feedback loop 54 and an ultrasonic feedback pump UFP, as previously described in connection with figure 2a, and a feeding pipe to allow organic material "A", "B" and "C", respectively, to be introduced into an inlet 55, 56 and 57 of the respective ultrasonic reactor US₁, US₂ and US₃ using feeding pumps PA, PB and PC, respectively. Ultrasonic treated organic material from an outlet 61 of the first ultrasonic reactor US₁ is transferred to the inlet 56 of the second ultrasonic reactor US₂ through feedback pipe 58, and ultrasonic treated organic material from an outlet 62 the second ultrasonic reactor US₂ is transferred to the inlet 57 of the third ultrasonic reactor US₃ through feedback pipe 59. A first and a second feed forward pipe 60, 65 are provided from the outlet 62 of the second ultrasonic reactor and an outlet 63 of the third ultrasonic reactor, respectively, to the outlet 61 of the first ultrasonic reactor US₁ in order to transfer ultrasonic treated organic material to an outflow 64 connected to the outlet 61 of the first ultrasonic reactor US₁.

The capacity of the ultrasonic feedback pumps UFP is preferably 15-30 m³/h and the capacity of the feeding pumps PA, PB and PC is preferably 0-15 m³/h. Thus, the flow through the feedback loop 54 is at least equal to the flow through each feeding pump, and more preferably twice as high or more. Feedback loop pumps FLP are provided in each feedback pipe 58 and 59, and the capacity of the FLP is preferably 0-15 m³/h.

Organic material having higher dry solid contents may be fed into the ultrasonic reactors compared to the embodiment described in connection with figure 4 due to the presence of the ultrasonic feedback loop.

Figure 6 shows a first embodiment of a biogas producing system 70 including a reactor tank 71 and disintegrating systems 72 and 73, such as described in connection with figures 3-5, in a feedback loop and pre-treatment for treatment of organic material in connection with producing biogas. It should be noted that the biogas producing system may be implemented with only a feedback loop or a pre-treatment system, as well as a combination of both.

The feedback loop comprises a first disintegrating system 72, similar to the system described in connection with figure 3 with the addition of an optional feedback pipe 74 from the outlet 76 of the reactor tank 71 to the pre-treatment of the organic material. A viscosity sensor 75 is also used to control the pumps within the disintegrating system 72. Additional organic material from other processes may be introduced as indicated in the drawing. If the feedback loop is implemented without pre-treatment of the organic material, the feedback pipe 74 is not needed.

The pre-treatment system comprises a second disintegrating system 73, similar to the system described in connection with figure 4 or 5 (if the optional feedback pipe 74 is present). Organic material A and B are introduced and treated (using mechanical dispersion or ultrasound as described above) within the disintegrating system 73, and material C from the outlet 76 is introduced to the disintegrating system. A main pump MP feeds an appropriate amount of treated material into the reactor tank 71. A viscosity sensor 79 and control unit CU within the disintegrating system controls the pump activity within the disintegrating system 73, and optionally also the FP pump controlling the amount of material in the feedback pipe 74, in order to output organic material with a desired viscosity to the tank reactor 71.

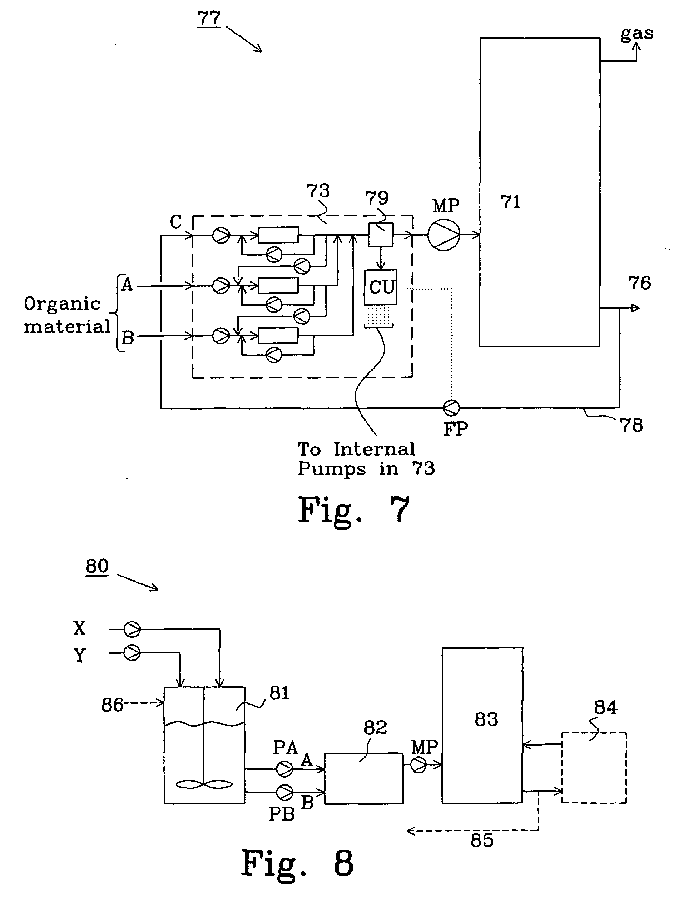

Figure 7 shows a second embodiment of a biogas producing system 77, including a reactor tank 71 and a disintegrating system 73, similar to the system disclosed in connection with figure 5.

The system comprises a feedback loop 78 from the outflow 76 of the tank reactor 71. The flow is controlled by a feedback pump FP and introduced into the first disintegrating unit in the disintegrating system 73. The purpose for feeding sludge from the tank reactor 71 to the first disintegrating unit is to provide organic material with a suitable viscosity in the system if other organic material A and B, which are introduced to the second and third disintegrating unit, have a viscosity that make them difficult to move through the respective disintegrating unit by themselves.

The disintegrating system preferably comprises a viscosity sensor 79 and a control unit configured to control the internal pumps in the disintegrating unit as well as the feedback pump FP.

Figure 8 shows an alternative embodiment of a biogas producing system 80 in which material "X" and "Y" with different properties, such as different temperature or dry solids contents, are mixed in a premixing vessel 81 before introduced into a disintegrating system 82. In this example two feed pipes with pumps PA and PB are used to introduce organic material "A" and "B" into the disintegrating system 82.

The organic material "A" and "B" are in this case the same and consists of a mixture of "X" and "Y". Optionally, nutriments may also be introduced into the premixing vessel 81 as indicated by the dashed arrow 86. A main pump MP will feed treated material from the disintegrating system 82 into a tank reactor 83.

It is also possible to add a feedback loop 84, as described in connection with figure 6, to the system in figure 7. A feedback pipe 85 may be implemented to feed material from an outlet of the tank reactor 83 either to the disintegrating system (as described in connection with figure 6) or as one of the materials "Y" introduced into the premixing vessel 81. "X" in this case would be non-treated organic material may have a different temperature, or dry solids contents, compared to the material obtained from the outlet of the tank reactor 83. The premixing vessel 81 will equalize any difference and might increase the efficiency of the disintegrating system.

In an alternative embodiment, the disintegrating system 82 may include a prior art disintegrating system, as disclosed in JP 2006-122875 and described in connection with figure 2a and 2b, with the addition of a viscosity detector, similar to that described in connection with figure 3 or 4, arranged at the outflow of the disintegrating system 82. A control unit attached to the viscosity detector will control the amount of organic material fed back to the inlet of the disintegrating unit (e.g. ultrasonic reactor) and thereby also the viscosity of the organic material available to be introduced into the reactor tank 83. In this case, only one inlet of organic material, e.g. "A", from the pre-mixing tank 81 is necessary.

Although the introduced material has been exemplified in connection with figures 3-7 as organic material "A", "B" and "C", which is generally preferred, the scope of the invention should not be limited to this since it might be desirable to introduce non-organic material into the system. However, in order to produce disintegrated organic material, at least one of the materials introduced has to be organic, and nutriments that could be used in a digestion process, such as Iron, Cobalt, Nickel, Selenium, Tungsten, Boron, Molybdenum, and Vanadium, may be introduced to be mixed with the organic material in the system, or the nutriments may be pre-mixed with organic material before it is introduced.

The pumps used in all the described embodiments of the invention are preferably eccentric screw pumps or chopper pumps, obtainable from e.g. ITT Flygt, KSB or Scanpump.

The terminology "disintegrating unit" is used in the claims as a generic term for ultrasonic reactor, mechanical dispersion device, as well as any other type of device that will disintegrate a material.

Furthermore, the disintegrating units of the described disintegrating units in connection with figures 3-8 are of the same type, i.e. either ultrasound reactors or mechanical dispersion devices, but the invention should not be limited to this. It is possible to construct a disintegrating system having a mechanical dispersion device as a first integrating unit and an ultrasound reactor as a second disintegrating system.

The capacity and operating characteristics of each pump is selected to transport the desired amount of organic material through each ultrasonic reactor, and to create a non-laminar flow through each ultrasonic reactor. It is preferred that the flow through each ultrasonic reactor is above 0.5 m/s in order to avoid an undesired laminar flow.

It should be noted that although the described embodiment above illustrates a biogas producing system having a feedback loop of treated digested sludge being fed back to the same tank reactor, as described in connection with figure 6, other configurations of tank reactors are possible. Tank reactors may be arranged in parallel and/or in series with each other, whereby digested sludge from a first tank reactor may be pumped into a disintegrating system and the treated digested sludge is thereafter delivered to a second tank reactor and/or delivered back to the first tank reactor.

## Claims

1. A disintegrating system for treatment of organic material comprising:
multiple disintegrating units (31, 32; 41, 42; 51, 52, 53), said disintegrating units are ultrasonic reactors and/or mechanical dispersion devices, each having an inlet (33, 34; 43, 44; 55, 56, 57) for receiving material (A, B, C), and an outlet (37, 38; 47, 48; 61, 62, 63) for outputting treated material, an inlet (33; 43; 55) of a first of said multiple disintegrating units (31; 41; 51) is configured to receive organic material (A),
a first feedback pipe (35; 45; 58, 59) connected between the outlet (37; 47; 61) of the first disintegrating unit and the inlet (34; 44; 56) of a second disintegrating unit of said multiple disintegrating units, and
an outflow (39; 49; 64) of said disintegrating system (30; 40; 50) is connected to the outlet (37; 47; 61) of at least the first disintegrating unit (31; 41; 51), wherein the sum of the introduced material (A, B, C) is available at the outflow (39; 49; 64), **characterized by**:
a viscosity detector (31; 66) provided to determine the viscosity of the treated material at the outlet of the first disintegrating unit, and
a control unit (65; 68) configured to receive measured parameters from the viscosity detector (31; 66) to control the flow through the first feedback pipe (35; 45; 58, 59).

2. The disintegrating system according to claim 1, wherein a second feedback pipe (36) is connected between the outlet (38) of the second disintegrating unit and the inlet (33) of the first disintegrating unit, said outflow (39) only is connected to the outlet (37) of the first disintegrating unit (31).

3. The disintegrating system according to claim 2, wherein a first feeding pump is provided to feed organic material (A) to the inlet (33) of the first disintegrating unit (31) and a second feeding pump is provided to feed material (B) to the inlet (34) of the second disintegrating unit (32), and a feedback loop pump is provided in the feedback pipe, the flow capacity of the feedback loop pump is at least equal to the flow capacity of each feeding pump.

4. The disintegrating system according to claim 1, wherein a feed forward pipe (46; 60) is connected between the outlet (48; 62, 63) of the second disintegrating unit and the outlet (47; 61) of the first disintegrating unit, said outflow (49; 64) is connected to the outlet (47, 48; 61, 62, 63) of multiple disintegrating units (41, 42; 51, 52, 53).

5. The disintegrating system according to claim 4, wherein a second feedback pipe (59) is connected between the outlet (62) of the second disintegrating unit and the inlet (57) of a third disintegrating unit, and a second feed forward pipe (65) between the outlet (63) of the third disintegrating unit and the outlet (61) of the first disintegrating unit.

6. The disintegrating system according to any of claims 1-5, wherein said viscosity detector is one of the group: a viscosity sensor (67) placed at the outflow (49); or an analyzer provided with voltage and current supplied to the first disintegrating unit (31).

7. A biogas producing system using anaerobic digestion of organic matter, said system comprising:
a tank reactor (71; 83) provided with an inlet for receiving organic material suitable for biogas production, said tank reactor containing biogas-producing bacteria for digestion under anaerobic conditions,
feeding means to feed said organic material into the tank reactor to obtain digestion while producing biogas and forming digested sludge, **characterized in that** said system further comprises:
at least one disintegrating system (72, 73; 82) according to any of claims 1-6, configured to feed treated organic material to said inlet.

8. A method for treatment of organic material in a disintegrating system (30; 40; 50) comprising multiple disintegrating units (31, 32; 41, 42; 51, 52, 53), each having an inlet (33, 34; 43, 44; 55, 56, 57) for receiving material (A, B, C), and an outlet (37, 38; 47, 48; 61, 62, 63) for outputting treated material, said method comprises:
providing said disintegrating units as ultrasonic reactors, and/or mechanical dispersion devices,
introducing organic material (A) at the inlet (33; 43; 55) of a first of said multiple disintegrating units (31; 41; 51),
providing treated organic material from at least the outlet of the first of said multiple disintegrating units (31; 41; 51) to an outflow (39; 49; 64) of said disintegrating system (30; 40; 50),
connecting a first feedback pipe (35; 45; 58, 59) between the outlet (37; 47; 61) of the first disintegrating unit and the inlet (34; 44; 56) of a second disintegrating unit of said multiple disintegrating units, and
providing the sum of the introduced material (A, B, C) at the outflow (39; 49; 64), **characterized by**:
providing a viscosity detector (31; 66) to determine the viscosity of the treated material at the outlet of the first disintegrating unit, and
configuring a control unit (65; 68) to receive measured parameters from the viscosity detector to control the flow through the first feedback pipe.

9. The method according to claim 8, wherein the method further comprises connecting a second feedback pipe (36) between the outlet (38) of the second disintegrating unit and the inlet (33) of the first disintegrating unit, and connecting only said outflow (39) to the outlet (37) of the first disintegrating unit (31).

10. The method according to claim 9, wherein the method further comprises:
providing a first feeding pump to feed organic material (A) to the inlet (33) of the first disintegrating unit (31), and
providing a second feeding pump to feed material (B) to the inlet (34) of the second disintegrating unit (32), and
providing a feedback loop pump in the second feedback pipe, and selecting the flow capacity of the feedback loop pump to be at least equal to the flow capacity of each feeding pump.

11. The method according to claim 8, wherein the method further comprises connecting a feed forward pipe (46; 60) between the outlet (48; 62, 63) of the second disintegrating unit and the outlet (47; 61) of the first disintegrating unit, and connecting said outflow (49; 64) to the outlet (47, 48; 61, 62, 63) of multiple disintegrating units (41, 42; 51, 52, 53).

12. The method according to claim 11, wherein the method further comprises connecting a second feedback pipe (59) between the outlet (62) of the second disintegrating unit and the inlet (57) of a third disintegrating unit, and connecting a second feed forward pipe (65) between the outlet (63) of the third disintegrating unit and the outlet (61) of the first disintegrating unit.

13. The method according to any of claims 8-12, wherein the method further comprises selecting said viscosity detector to be one of the group: a viscosity sensor (67) placed at the outflow (49); or an analyzer provided with voltage and current supplied to the first disintegrating unit (31).

## Patentansprüche

1. Zerkleinerungssystem zur Behandlung von organischem Material, umfassend:
viele Zerkleinerungseinheiten (31, 32; 41, 42; 51, 52, 53), wobei die Zerkleinerungseinheiten Ultraschallreaktoren und/oder mechanische Zerteilungsvorrichtungen sind, von denen jede einen Einlass (33, 34; 43, 44; 55, 56, 57) zum Aufnehmen von Material (A, B, C) und einen Auslass (37, 38; 47, 48; 61, 62, 63) zum Ausgeben des behandelten Materials aufweist, wobei ein Einlass (33; 43; 55) einer ersten der vielen Zerkleinerungseinheiten (31; 41; 51) ausgelegt ist, organisches Material (A) aufzunehmen,
ein erstes Rücklaufrohr (35; 45; 58; 59), das zwischen dem Auslass (37; 47; 61) der ersten Zerkleinerungseinheit und dem Einlass (34; 44; 56) einer zweiten Zerkleinerungseinheit der vielen Zerkleinerungseinheiten angeordnet ist; und
einen Auslauf (39; 49; 64) des Zerkteinerungssystems (30; 40; 50), der mit dem Auslass (37; 47; 61) zumindest der ersten Zerkleinerungseinheit (31; 41; 51) verbunden ist, wobei die Summe des eingeleiteten Materials (A, B, C) am Auslauf (39; 49; 64) verfügbar ist, **gekennzeichnet durch**:
einen Viskositätsdetektor (31; 66), der vorgesehen ist, um die Viskosität des behandelten Materials am Auslass der ersten Zerkleinerungseinheit zu bestimmen, und
eine Steuereinheit (65; 68), die ausgelegt ist, gemessene Parameter vom Viskositätsdetektor (31; 66) zu empfangen, und den Durchfluss **durch** das erste Rücklaufrohr (45; 45; 58; 59) zu steuern.

2. Zerkleinerungssystem nach Anspruch 1, wobei ein zweites Rücklaufrohr (36) zwischen dem Auslass (38) der zweiten Zerkleinerungseinheit und dem Einlass (33) der ersten Zerkleinerungseinheit angeordnet ist, wobei der Auslauf (39) nur mit dem Auslass (37) der ersten Zerkleinerungseinheit (31) verbunden ist.

3. Zerkleinerungssystem nach Anspruch 2, wobei eine erste Speisepumpe vorgesehen ist, um organisches Material (A) zum Einlass (33) der ersten Zerkleinerungseinheit (31) zuzuführen, und eine zweite Speisepumpe vorgesehen ist, um Material (B) zum Einlass (34) der zweiten Zerkleinerungseinheit (32) zuzuführen, und eine Rückführkreispumpe in dem Rücklaufrohr vorgesehen ist, wobei die Durchflusskapazität der Rückführkreispumpe zumindest gleich der Durchflusskapazität jeder Speisepumpe ist.

4. Zerkleinerungssystem nach Anspruch 1, wobei ein Zulaufrohr (46; 60) zwischen dem Auslass (48; 62; 63) der zweiten Zerkleinerungseinheit und dem Auslas (47; 61) der ersten Zerkleinerungseinheit angeordnet ist, wobei der Auslauf (49; 64) mit dem Auslass (47, 48; 61, 62, 63) der vielen Zerkleinerungseinheiten (41, 42; 51, 52, 53) verbunden ist.

5. Zerkleinerungssystem nach Anspruch 4, wobei ein zweites Rücklaufrohr (59) zwischen dem Auslass (62) der zweiten Zerkleinerungseinheit und dem Einlass (57) einer dritten Zerkleinerungseinheit und ein zweites Zulaufrohr (65) zwischen dem Auslass (63) der dritten Zerkleinerungseinheit und dem Auslass (61) der ersten Zerkleinerungseinheit angeordnet ist.

6. Zerkleinerungssystem nach einem der Ansprüche 1 bis 5, wobei der Viskositätsdetektor ein Viskositätssensor (67), der am Auslauf (49) angeordnet ist, oder ein Messgerät, das mit Spannung und Strom, die zur ersten Zerkleinerungseinheit (31) zugeführt werden, versorgt wird, ist.

7. Biogaserzeugungssystem, das eine anaerobe Faulung von organischem Material nutzt, wobei das System umfasst:
einen Tankreaktor (71; 83), der mit einem Einlass zum Aufnehmen von organischem Material, das für die Biogaserzeugung geeignet ist, ausgestattet ist, wobei der Tankreaktor Biogas erzeugende Bakterien für die Faulung unter anaeroben Bedingungen enthält,
eine Zuführeinrichtung zum Zuführen des organischen Materials in den Tankreaktor, um die Faulung zu erzielen, während Biogas erzeugt und Faulschlamm gebildet wird,
**dadurch gekennzeichnet, dass** das System ferner umfasst:
zumindest ein Zerkleinerungssystem (72, 73; 82) nach einem der Ansprüche 1 bis 6, das ausgelegt ist, das behandelte organische Material zum Einlass zu transportieren.

8. Verfahren zur Behandlung von organischem Material in einem Zerkleinerungssystem (30; 40; 50), das viele Zerkleinerungseinheiten (31, 32; 41, 42; 51, 52, 53) umfasst, von denen jede einen Einlass (33, 34,; 43, 44; 55, 56, 57) zum Aufnehmen von Material (A, B, C) und einen Auslass (37, 38; 47, 48; 61, 62, 63) zum Ausgeben von behandeltem Material aufweist, wobei das Verfahren umfasst:
Bereitstellen der Zerkleinerungseinheiten als Ultraschallreaktoren und/oder mechanische Zerteilungsvorrichtungen,
Einleiten des organischen Materials (A) am Einlass (33; 43; 55) einer ersten der vielen Zerkleinerungseinheiten (31; 41; 51),
Bereitstellen des behandelten organischen Material von zumindest dem Auslass der ersten der vielen Zerkleinerungseinheiten (31; 41; 51) für einen Auslauf (39; 49; 64) des Zerkleinerungssystems (30; 40; 50),
Verbinden eines ersten Rücklaufrohrs (35; 45; 58, 509) zwischen dem Auslass (37; 47; 61) der ersten Zerkleinerungseinheit und dem Einlass (34; 44; 56) einer zweiten Zerkleinerungseinheit der vielen Zerkleinerungseinheiten, und
Bereitstellen der Summe des eingeleiteten Materials (A, B, C) am Auslauf (38; 49; 64), **gekennzeichnet durch**:
Bereitstellen eines Viskositätsdetektors (31; 66), um die Viskosität des behandelten Materials am Auslass der ersten Zerkleinerungseinheit zu bestimmen, und
Auslegen einer Steuereinheit (65; 68), gemessene Parameter vom Viskositätsdetektor zu empfangen, um den Durchfluss **durch** das erste Rücklaufrohr zu steuern.

9. Verfahren nach Anspruch 8, wobei das Verfahren ferner ein Verbinden eines zweiten Rücklaufrohrs (36) zwischen dem Auslass (38) der zweiten Zerkleinerungseinheit und dem Einlass (33) der ersten Zerkleinerungseinheit, und nur Verbinden des Auslaufs (39) mit dem Auslass (37) der ersten Zerkleinerungseinheit (31) umfasst.

10. Verfahren nach Anspruch 9, wobei das Verfahren ferner umfasst:
Bereitstellen einer ersten Speisepumpe, um organisches Material (A) zum Einlass (33) der ersten Zerkleinerungseinheit (31) zuzuführen, und
Bereitstellen eine zweiter Speisepumpe, um Material (B) zum Einlass (34) der zweiten Zerkleinerungseinheit (32) zuzuführen, und
Bereitstellen einer Rückführkreispumpe in dem zweiten Rücklaufrohr, und Auswählen der Durchflusskapazität der Rückführkreispumpe, damit diese zumindest gleich der Durchflusskapazität jeder Speisepumpe ist.

11. Verfahren nach Anspruch 8, wobei das Verfahren ferner ein Verbinden eines Zulaufrohrs (46; 60) zwischen dem Auslass (48; 62; 63) der zweiten Zerkleinerungseinheit und dem Auslas (47; 61) der ersten Zerkleinerungseinheit und ein Verbinden des Auslaufs (49; 64) mit dem Auslass (47, 48; 61, 62, 63) der vielen Zerkleinerungseinheiten (41, 42; 51, 52, 53) umfasst.

12. Verfahren nach Anspruch 11, wobei das Verfahren ferner ein Verbinden eines zweiten Rücklaufrohrs (59) zwischen dem Auslass (62) der zweiten Zerkleinerungseinheit und dem Einlass (57) einer dritten Zerkleinerungseinheit und Verbinden eines zweiten Zulaufrohrs (65) zwischen dem Auslass (63) der dritten Zerkleinerungseinheit und dem Auslass (61) der ersten Zerkleinerungseinheit umfasst.

13. Verfahren nach einem der Ansprüche 8 bis 12, wobei das Verfahren ferner ein Auswählen des Viskositätsdetektors als ein Viskositätssensor (67), der am Auslauf (49) angeordnet ist, oder als ein Messgerät, das mit Spannung und Strom, die zur ersten Zerkleinerungseinheit (31) zugeführt werden, versorgt wird, umfasst.

## Revendications

1. Système de désintégration pour le traitement de matière organique comprenant :
des unités de désintégration multiples (31, 32 ; 41, 42 ; 51, 52, 53), lesdites unités de désintégration sont des réacteurs à ultrasons et/ou des dispositifs de dispersion mécanique, comportant chacune une entrée (33, 34 ; 43, 44 ; 55, 56, 57) destinée à recevoir la matière (A, B, C) et une sortie (37, 38 ; 47, 48 ; 61, 62, 63) destinée à produire en sortie la matière traitée, une entrée (33 ; 43 ; 55) d'une première desdites unités de désintégration multiples (31 ; 41 ; 51) est configurée pour recevoir la matière organique (A),
une première conduite à rétroaction (35 ; 45 ; 58, 59) raccordée entre la sortie (37 ; 47 ; 61) de la première unité de désintégration et l'entrée (34 ; 44 ; 56) d'une deuxième unité de désintégration desdites unités de désintégration multiples, et
un écoulement (39 ; 49 ; 64) dudit système de désintégration (30 ; 40 ; 50) est raccordé à la sortie (37 ; 47 ; 61) d'au moins la première unité de désintégration (31 ; 41 ; 51), dans lequel la somme de la matière introduite (A, B, C) est disponible au niveau de l'écoulement (39 ; 49 ; 64), **caractérisé par** :
un détecteur de viscosité (31 ; 66) prévu pour déterminer la viscosité de la matière traitée au niveau de l'écoulement de la première unité de désintégration, et
une unité de commande (65 ; 68) configurée pour recevoir des paramètres mesurés en provenant du détecteur de viscosité (31 ; 66) afin de commander l'écoulement à travers la première conduite à rétroaction (35 ; 45 ; 58, 59).

2. Système de désintégration selon la revendication 1, dans lequel une seconde conduite à rétroaction (36) est raccordée entre la sortie (38) de la deuxième unité de désintégration et l'entrée (33) de la première unité de désintégration, seul ledit écoulement (39) est raccordé à la sortie (37) de la première unité de désintégration (31).

3. Système de désintégration selon la revendication 2, dans lequel une première pompe d'alimentation est prévue pour alimenter l'entrée (33) de la première unité de désintégration (31) en matière organique (A) et une seconde pompe d'alimentation est prévue pour alimenter l'entrée (34) de la deuxième unité de désintégration (32) en matière (B), et une pompe de boucle de rétroaction est prévue dans la conduite de rétroaction, le pouvoir d'écoulement de la pompe de boucle de rétroaction est au moins égal au pouvoir d'écoulement de chaque pompe d'alimentation.

4. Système de désintégration selon la revendication 1, dans lequel une conduite d'action directe (46 ; 60) est raccordée entre la sortie (48 ; 62, 63) de la deuxième unité de désintégration et la sortie (47 ; 61) de la première unité de désintégration, ledit écoulement (49 ; 64) est raccordé à la sortie (47, 48 ; 61, 62, 63) des unités de désintégration multiples (41, 42 ; 51, 52, 53).

5. Système de désintégration selon la revendication 4, dans lequel une seconde conduite de rétroaction (59) est raccordée entre la sortie (62) de la deuxième unité de désintégration et l'entrée (57) d'une troisième unité de désintégration, et une seconde conduite d'action directe (65) entre la sortie (63) de la troisième unité de désintégration et la sortie (61) de la première unité de désintégration.

6. Système de désintégration selon l'une quelconque des revendications 1 à 5, dans lequel ledit détecteur de viscosité est un élément du groupe : un capteur de viscosité (67) placé au niveau de l'écoulement (49) ; ou un analyseur pourvu de tension et de courant fournis à la première unité de désintégration (31).

7. Système de production de biogaz utilisant une digestion anaérobie de matière organique, ledit système comprenant :
un réacteur-cuve (71 ; 83) pourvu d'une entrée destinée à recevoir la matière organique adaptée pour la production de biogaz, ledit réacteur-cuve contenant des bactéries productrices de biogaz pour une digestion dans des conditions anaérobies,
des moyens d'alimentation afin d'alimenter le réacteur-cuve en ladite matière organique pour obtenir une digestion tout en produisant du biogaz et en formant des boues digérées,
**caractérisé en ce que** ledit système comprend en outre :
au moins un système de désintégration (72, 73 ; 82) selon l'une quelconque des revendications 1 à 6, configuré pour alimenter ladite entrée en matière organique traitée.

8. Procédé de traitement de matière organique dans un système de désintégration (30 ; 40 ; 50) comprenant des unités de désintégration multiples (31, 32 ; 41, 42 ; 51, 52, 53), comportant chacune une entrée (33, 34 ; 43, 44 ; 55, 56, 57) destinée à recevoir la matière (A, B, C) et une sortie (37, 38 ; 47, 48 ; 61, 62, 63) destinée à produire en sortie la matière traitée, ledit procédé comprenant les étapes consistant à :
fournir lesdites unités de désintégration multiples en tant que réacteurs à ultrasons, et/ou dispositifs de dispersion mécanique,
introduire la matière organique (A) au niveau de l'entrée (33 ; 43 ; 55) d'une première desdites unités de désintégration multiples (31 ; 41 ; 51),
fournir la matière organique traitée en provenance d'au moins la sortie de la première desdites unités de désintégration multiples (31 ; 41 ; 51) à un écoulement (39 ; 49 ; 64) dudit système de désintégration (30 ; 40 ; 50),
raccorder une première conduite de rétroaction (35 ; 45 ; 58, 59) entre la sortie (37 ; 47 ; 61) de la première unité de désintégration et l'entrée (34 ; 44 ; 56) d'une deuxième unité de désintégration desdites unités de désintégration multiples, et
fournir la somme de la matière introduite (A, B, C) à l'écoulement (39 ; 49 ; 64), **caractérisé par** les étapes consistant à :
fournir un détecteur de viscosité (31 ; 66) afin de déterminer la viscosité de la matière traitée à la sortie de la première unité de désintégration, et
configurer une unité de commande (65 ; 68) afin de recevoir des paramètres mesurés du détecteur de viscosité afin de commander l'écoulement à travers la première conduite à rétroaction.

9. Procédé selon la revendication 8, dans lequel le procédé comprend en outre les étapes consistant à raccorder une seconde conduite de rétroaction (36) entre la sortie (38) de la deuxième unité de désintégration et l'entrée (33) de la première unité de désintégration, et raccorder seulement ledit écoulement (39) à la sortie (37) de la première unité de désintégration (31).

10. Procédé selon la revendication 9, dans lequel le procédé comprend en outre les étapes consistant à :
fournir une première pompe d'alimentation pour alimenter l'entrée (33) de la première unité de désintégration (31) en matière organique (A), et
fournir une seconde pompe d'alimentation pour alimenter l'entrée (34) de la deuxième unité de désintégration (32) en matière (B), et
fournir une pompe de boucle de rétroaction dans la seconde conduite à rétroaction, et sélectionner le pouvoir d'écoulement de la pompe de boucle de rétroaction pour qu'il soit au moins égal au pouvoir d'écoulement de chaque pompe d'alimentation.

11. Procédé selon la revendication 8, dans lequel le procédé comprend en outre les étapes consistant à raccorder une première conduite à action directe (46 ; 60) entre la sortie (48 ; 62, 63) de la deuxième unité de désintégration et la sortie (47 ; 61) de la première unité de désintégration, et raccorder ledit écoulement (49 ; 64) à la sortie (47, 48 ; 61, 62, 63) des unités de désintégration multiples (41, 42 ; 51, 52, 53).

12. Procédé selon la revendication 11, dans lequel le procédé comprend en outre les étapes consistant à raccorder une seconde pompe de rétroaction (59) entre la sortie (62) de la deuxième unité de désintégration et l'entrée (57) d'une troisième unité de désintégration, et raccorder une seconde conduite à action directe (65) entre la sortie (63) de la troisième unité de désintégration et la sortie (61) de la première unité de désintégration.

13. Procédé selon l'une quelconque des revendications 8 à 12, dans lequel le procédé comprend en outre l'étape consistant à sélectionner ledit détecteur de viscosité pour qu'il soit l'un du groupe : un capteur de viscosité (67) placé au niveau de l'écoulement (49) ; ou un analyseur pourvu de tension et de courant fournis à la première unité de désintégration (31).
